# EUROPEAN PATENT APPLICATION

(11) **EP 2 322 172 A1**
(43) Date of publication of application: **18.05.2011**
(21) Application number: 09290827.6
(22) Date of filing: 29.10.2009
(51) Int. Cl.: A61K 31/343, A61P 9/06

(54) **Use of celivarone for preparing a medicament for the treatment of patients with arrhythmia and having an increase of creatinine level due to celivarone administration**

(71) Applicant: Sanofi-Aventis, 75013 Paris (FR)
(72) Inventor: Bergougnan, Luc, 75013 Paris (FR); Radzic, Davide, 75013 Paris (FR)
(74) Representative: Romanowski, Caroline

(57) **Abstract**

Use of celivarone or one of its pharmaceutically acceptable salts for preparing a medicament for the treatment of patients with arrhythmia, said patients having an increase of creatinine level due to celivarone administration, said creatinine level increasing following celivarone treatment initiation, said creatinine level increase reaching a plateau and being used as a new baseline, said creatinine level increase being reversible after celivarone discontinuation.

## Description

The instant invention relates to a use of celivarone or one of its pharmaceutically acceptable salts for preparing a medicament for the treatment of patients with arrhythmia, wherein said patients have an increase of creatinine level due to celivarone administration and to a method of managing the risk due to an observation of serum creatinine increase leading to inappropriate management of these patients. The instant invention more specifically relates to a method of managing the risk due to an observation of serum creatinine increase in patients treated by celivarone and by Angiotensin Converting Enzyme Inhibitors (ACE-I) or Angiotensin Receptor Blockers (ARB) or potassium sparing diuretics.

The antiarrhythmic agent isopropyl 2-butyl-3-{4-[3-dibutylamino) propyl]benzoyl}-1-benzofuran-5 carboxylate fumarate, or celivarone, and its other pharmaceutically acceptable salts are described in the European patent EP 0 315 709.

Creatinine is an end-product of muscle metabolism, which is freely filtered at the glomerulus and not metabolized in the kidney. It is secreted by the proximal tubules by both the anionic and the cationic secretory pathways. Usually an increase in creatinine level is considered as a marker of decreased glomerular filtration and as a sign of renal impairment (Journal of Internal medicine, 1999, 246, 247-252).

During clinical trials with celivarone, it has been observed that administration of celivarone may be associated with a moderate increase in creatinine serum levels of the patient. It has however been demonstrated that such an increase in the creatinine levels is not linked to renal impairment, but is due to decreased secretion of this substance at the kidney tubular level. In fact, in a specific study in healthy subjects, this increase was shown to be related to inhibition of creatinine secretion at the tubular level, with no effect on glomerular filtration or on renal blood flow. Hence celivarone can not be considered as a nephrotoxic agent. This decrease in the tubular secretion of creatinine is also reported with other drugs such as cimetidine, trimethoprim or even amiodarone (Br. J. Clin. Pharmac., 1993, 36, 125-127).

This effect of celivarone on the creatinine levels becomes of particular relevance when this agent is co-prescribed to patients receiving another therapy that could interact with kidney function. This is especially the case for the treatment of patients with arrhythmia or other cardiovascular diseases, such as patients with heart failure, coronary disease and other cardiovascular risk factors receiving ACE-I and/or ARB and/or spironolactone or other potassium sparing diuretics. Indeed, such categories of active ingredients are known for their adverse effects on the renal function. In patients treated both by celivarone and by ACE-I and/or ARB and/or potassium sparing diuretics, an increase in creatinine levels might be misinterpreted by the physician as a sign of nephrotoxicity of ACE-I, ARB or potassium sparing diuretics, leading to inappropriate discontinuation of these agents. These treatments have been shown to prevent mortality in patients with heart failure (Pfeffer MA et al., Effect of captopril on mortality and morbidity in patients with left ventricular dysfunction after myocardial infarction. Results of the survival and ventricular enlargement trial. The SAVE Investigators, N. Engl. J. Med. 1992 Sep 3;327(10):669-77; Pitt B et al., The effect of spironolactone on morbidity and mortality in patients with severe heart failure. Randomized Aldactone Evaluation Study Investigators, N. Engl. J. Med. 1999;341:709-717; Pitt B et al., Eplerenone, a selective aldosterone blocker, in patients with left ventricular dysfunction after myocardial infarction., N. Engl. J. Med. 2003;348:1309-1321) and in patients with coronary disease (Indications for ACE inhibitors in the early treatment of acute myocardial infarction: systematic overview of individual data from 100,000 patients in randomized trials. ACE Inhibitor Myocardial Infarction Collaborative Group. Circulation 1998;97:2202-2212; Swedberg K et al., Effects of the early administration of enalapril on mortality in patients with acute myocardial infarction. Results of the Cooperative New Scandinavian Enalapril Survival Study II (CONSENSUS II)., N. Engl. J. Med. 1992;327:678-684; Dickstein K et al., Effects of losartan and captopril on mortality and morbidity in high-risk patients after acute myocardial infarction: the OPTIMAAL randomised trial. Optimal Trial in Myocardial Infarction with Angiotensin II Antagonist Losartan, Lancet 2002;360:752-760) or to prevent events, including cardiovascular mortality, in patients at cardiovascular risk (Yusuf S et al., Effects of an angiotensin-converting-enzyme inhibitor, ramipril, on cardiovascular events in high-risk patients. The Heart Outcomes Prevention Evaluation Study Investigators., N. Engl. J. Med. 2000 Jan 20;342(3):145-53). Inappropriate interruption of the treatment with ACE-I, ARB or potassium sparing diuretics therefore exposes these patients to an increased cardiovascular morbidity and mortality.

The Applicant has now found a method for managing such a risk. The method according to the invention enables to decrease the risk of an inappropriate interruption of ACE-I or ARB treatment or of potassium sparing diuretics, which consequently enables to decrease the risk of morbidity and/or mortality of the patient.

Indeed, the effect of celivarone 300 mg once a day for 7 days on renal function in healthy male subjects was assessed in a randomized, double-blind, placebo-controlled, 2-way crossover clinical study (PDY5860). The primary objective was to evaluate the effect of repeated doses of celivarone on the renal creatinine clearance and on the glomerular filtration rate (estimated by renal sinistrin clearance) compared with placebo in healthy male subjects.

Results of this study have demonstrated that celivarone, at the dose regimen of 300 mg once a day for 7 days, reduced creatinine clearance by 7% to 8% (-7.53%, p=0.005), with no statistically significant difference in renal sinistrin clearance. A slight concomitant increase in plasma creatinine concentration is observed compared to placebo (+2.8% on average on Day 7). A concomitant and significant decrease in renal N¹-methylnicotinamide clearance by 13% suggests that celivarone partially inhibits organic cation transport, thus reducing tubular creatinine secretion, but does not mean a decline in renal function. Creatinine and N¹-methylnicotinamide clearances returned to baseline values at the recovery period (after discontinuation of celivarone treatment).

The instant invention therefore relates to a method of managing the risk of an increased morbidity and/or mortality rate in patients treated by an association of celivarone or one of its pharmaceutically acceptable salts and of at least a compound B selected from ACE-I, ARB and potassium sparing diuretics. Said method is carried out by performing the following steps:
a) initially measuring serum creatinine levels, such as within one week after initiation of treatment with celivarone, which provides a reference level,
b) monitoring serum creatinine levels at regular intervals during treatment of the patient by the compound B (as recommended in the labelling of compound B),
c) if the serum creatinine level increases above the reference level, clinical judgement should be made to determine if the increase is due to celivarone, to compound B or to another cause,
d) to help clinical judgment and evaluate if the increase is due to celivarone, celivarone can be temporarily interrupted. The return to baseline within about one week will be highly suggestive of celivarone responsibility. If despite discontinuation, creatinine levels do not decrease, another cause including other treatments taken by the patient should be considered.
   - If, by this mean, the increase is thought to be due to celivarone, concomitant treatment with B can be pursued with regular monitoring of creatinine levels,
   - if the increase in the serum creatinine level above the reference level is thought to be due to compound B, then labelling instructions of compound B should be followed and alternative treatment could be considered,
   - if the increase in the serum creatinine level above the reference level is due to another cause than administration of celivarone or of compound B, then appropriate treatment of such cause shall be undertaken,
e) repeating steps b), c) and d) for the duration of the treatment with celivarone and compound B.

The instant invention further relates to a method of managing the risk of an increase in the morbidity and/or mortality rate in patients treated by an association of celivarone or one of its pharmaceutically acceptable salts and of at least a compound B selected from ACE-I, ARB and potassium sparing diuretics, which method comprises the following steps:
a) initially measuring serum creatinine levels, which provides a reference level,
b) monitoring serum creatinine levels at regular intervals during treatment of the patient by celivarone and by the compound B,
c) if the serum creatinine level increases above the reference level, determining if the increase is due to celivarone, to compound B or to another cause,
d) if the increase in the serum creatinine level above the reference level is due to celivarone, then the treatment with celivarone and compound B can be pursued; if the increase in the serum creatinine level above the reference level is due to compound B, then the treatment with compound B may be interrupted; if the increase in the serum creatinine level above the reference level is due to another cause than administration of celivarone or of compound B, then appropriate treatment of such another cause shall be undertaken,
e) repeating steps b), c) and d) for the duration of the treatment with celivarone and compound B.

The instant invention further relates to a method of treatment of patients with cardiac arrhythmia comprising the administration of celivarone or one of its pharmaceutically acceptable salts to patients treated, in addition, by at least a compound B selected from ACE-I, ARB and potassium sparing diuretics, which method comprises the following steps:
a) initially measuring serum creatinine levels, which provides a reference level,
b) monitoring serum creatinine levels at regular intervals during treatment of the patient by celivarone and by the compound B,
c) if the serum creatinine level increases above the reference level, determining if the increase is due to celivarone, to compound B or to another cause,
d) if the increase in the serum creatinine level above the reference level is due to celivarone, then the treatment with celivarone and compound B can be pursued; if the increase in the serum creatinine level above the reference level is due to compound B, then the treatment with compound B may be interrupted; if the increase in the serum creatinine level above the reference level is due to another cause than administration of celivarone or of compound B, then appropriate treatment of such another cause shall be undertaken,
e) repeating steps b), c) and d) for the duration of the treatment with celivarone and compound B.

Examples of ACE Inhibitors may be captopril, enalapril, perindopril, quinapril, lisinopril, ramipril, etc ...

Examples of Angiotensin Receptor Blockers may be losartan, valsartan, candesartan, telmisartan, irbesartan, etc ...

Examples of potassium sparing diuretics may be spironolactone, eplerenone, etc ...

Such compounds are usually prescribed for prevention and treatment of various pathologies of the cardiac function, such as cardiovascular disorders, congestive heart failure, left ventricular dysfunction or hypertension.

Concerning step a) of the methods of the invention, as mentioned before, in most patients with arrhythmia the mean increase in plasma creatinine concentration, compared to the level before administration of celivarone, was 2.8%. Then during the treatment with celivarone, typically between 3 to 11 days, mean creatinine levels remain stable, corresponding to a plateau.

In step a) of the methods according to the invention, the serum creatinine levels may in particular be measured one week after the start of celivarone administration to the patient.

By "initially" in step a), one may for instance mean "one week after initiation of treatment with celivarone", being understood that a few days less or more are also encompassed within the scope of the step a), so that the reference level for serum creatinine may be measured at 3 to 15 days, more particularly 3 to 11 days, for example, after start of celivarone administration to the patient.

In step b) of the methods according to the invention, the monitoring of the serum creatinine levels at regular intervals means that the serum creatinine shall be measured over the total duration of the treatment with celivarone and compound B, based on a schedule depending on the specific pathological state of the patient and on the prescription labelling for the compound B. Such monitoring may for example be performed every 2 months in patients with renal impairment.

Step c) of the methods according to the instant invention targets to identify whether celivarone or compound B may be the cause of the increase in the creatinine level above the reference level, or if it may be due to another cause, i.e. an outside cause than the active principles administered to the patient. In fact, various pathological states can be responsible for transient or permanent creatinine elevation, for example diabetes or impaired cardiac function, deshydration, hypovolemia, renal impairment, drug toxicity etc ... Thus, a deterioration of the pathological state of the patient under the treatment of celivarone and of compound B may involve serum creatinine elevation.

Step c) may be achieved by temporarily interrupting treatment with celivarone, while maintaining treatment with compound B. Indeed, compound B should not be primarily interrupted unless there is a specific reason.

The temporary interruption of celivarone treatment or celivarone discontinuation may for example range from 1 to 2 weeks. Then the serum creatinine level shall be measured. If it returns to the reference level, then celivarone treatment shall be reinstated. Indeed, this would mean that the increase in creatinine was due to celivarone itself, and not related to any other concomitant disease including other drug toxicity.

On the other hand, if the creatinine level does not return to the reference level after the celivarone treatment interruption, then it would imply that celivarone is not the cause of the further creatinine increase. In such a case it shall be determined whether the increase is due to compound B, in which case treatment with compound B may be interrupted, or at least closely monitored to avoid renal impairment, or if the increase is due to another cause, as described above, in which case appropriate treatment of such other cause shall be undertaken. Appropriate treatment shall be appreciated by the physician depending on the patient's clinical condition and associated pathologies.

Another subject of the instant invention is the use of celivarone for preparing a medicament for use in the treatment of arrhythmia, wherein celivarone or one of its pharmaceutically acceptable salts is used in combination with a compound B selected from ACE-I, ARB and potassium sparing diuretics and wherein said use involves the following steps:
a) initially measuring serum creatinine levels, which provides a reference level,
b) monitoring serum creatinine levels at regular intervals during treatment of the patient by celivarone and by the compound B,
c) if the serum creatinine level increases above the reference level, determining if the increase is due to celivarone, to compound B or to another cause,
d) if the increase in the serum creatinine level above the reference level is due to celivarone, then the treatment with celivarone and compound B can be pursued; if the increase in the serum creatinine level above the reference level is due to compound B, then the treatment with compound B may be interrupted; if the increase in the serum creatinine level above the reference level is due to another cause than administration of celivarone or of compound B, then appropriate treatment of such another cause shall be undertaken,
e) repeating steps b), c) and d) for the duration of the treatment with celivarone and compound B.

In an embodiment, the subject of the instant invention is the use of an association of celivarone or one of its pharmaceutically acceptable salts and at least a compound B selected from ACE-I, ARB and potassium sparing diuretics for preparing a medicament for the treatment of patients with arrhythmia, said patients having an increase of creatinine level due to celivarone administration, said patients being defined with the following steps:
a) initially measuring serum creatinine levels, which provides a reference level,
b) monitoring serum creatinine levels at regular intervals during treatment of the patient by celivarone and by the compound B,
c₁) if the serum creatinine levels increases above the reference level, temporarily interrupting treatment with celivarone, while maintaining treatment with compound B
d₁) when the increase is reversible, treatment with said combination is pursued,
e₁) repeating steps b), c₁) and d₁) for the duration of the treatment with said combination.

In an embodiment, the subject of the instant invention is the use of an association of celivarone or one of its pharmaceutically acceptable salts and at least a compound B selected from ACE-I, ARB and potassium sparing diuretics for preparing a medicament for the treatment of patients with arrhythmia, said patients having an increase of creatinine level due to compound B administration, said patients being defined with the following steps:
a) initially measuring serum creatinine levels, which provides a reference level,
b) monitoring serum creatinine levels at regular intervals during treatment of the patient by celivarone and by the compound B,
c₁) if the serum creatinine levels increases above the reference level, temporarily interrupting treatment with celivarone, while maintaining treatment with compound B
d₂) when the increase is not reversible, treatment with compound B may be interrupted
e₂) repeating steps b), c₁) and d₂) for the duration of the treatment with said combination.

In an embodiment, the subject of the instant invention is the use of celivarone or one of its pharmaceutically acceptable salts for preparing a medicament for the treatment of patients with arrhythmia, said patients having an increase of creatinine level due to celivarone administration, said creatinine level increasing following celivarone treatment initiation, said creatinine level increase reaching a plateau and being used as a new baseline, said creatinine level increase being reversible after celivarone discontinuation.

In an embodiment, the subject of the instant invention is the use of celivarone or one of its pharmaceutically acceptable salts for preparing a medicament for the treatment of patients with arrhythmia, said patients having an increase of creatinine level due to celivarone administration, wherein said use involves the following steps:
i) initiating celivarone treatment
ii) measuring serum creatinine level increase following celivarone treatment initiation, which provides a reference level, said serum creatinine level reaching a plateau,
iii) discontinuing temporarily celivarone,
iv) measuring that the increase is reversible after celivarone discontinuation.

In an embodiment, the subject of the instant invention is the use of an association of celivarone or one of its pharmaceutically acceptable salts and compound B as defined above for preparing a medicament for the treatment of patients with arrhythmia, said patients having an increase of creatinine level not due to compound B administration, said creatinine level increasing following celivarone treatment initiation, said creatinine level increase reaching a plateau and being used as a new baseline, said creatinine level increase being reversible after celivarone discontinuation.

In an embodiment, the subject of the instant invention is the use of an association of celivarone or one of its pharmaceutically acceptable salts and compound B as defined above for preparing a medicament for the treatment of patients with arrhythmia, said patients having an increase of creatinine level not due to compound B administration, wherein said use involves the following steps:
i) initiating celivarone treatment
ii) measuring serum creatinine level increase following celivarone treatment initiation, which provides a reference level, said serum creatinine level reaching a plateau,
iii) discontinuing temporarily celivarone,
iv) measuring that the increase is reversible after celivarone discontinuation.

The above mentioned plateau may be reached after 3 to 15 days, more particularly 3 to 11 days, for example after about one week.

The fact that the creatinine level increase is reversible may be measured within about 1 week after celivarone discontinuation.

According to the instant invention:
- the expressions "reference level" and "new baseline" are equivalent;
- the plateau is defined by the measurement of serum creatinine level;
- serum creatinine level is measured from a blood sample, said blood sample being obtained from a patient.

In an embodiment, the subject of the instant invention is a method of treatment of patients with arrhythmia comprising:
i) initiating administration of celivarone or a pharmaceutically acceptable salt thereof in said patient,
ii) obtaining a blood sample from the patient following initiation of administration of celivarone, or a pharmaceutically acceptable salt thereof, to the patient, and
iii) measuring the serum creatinine levels from the blood sample to provide a reference level.

In some embodiments, the patient is also being treated with a compound selected from the group consisting of ACE-I, ARB and potassium sparing diuretics. In some embodiments, the serum creatinine level in step iii) is measured within 3 to 15 days, more particularly within 3 to 11 days after initiation of treatment with celivarone. In some embodiments, the serum creatinine level in step iii) is measured within one week after initiation of treatment with celivarone.

In an embodiment, the subject of the instant invention is a method of treatment of patients with arrhythmia comprising:
i) initiating administration of celivarone or a pharmaceutically acceptable salt thereof in said patient;
ii) obtaining a blood sample from the patient following initiation of administration of celivarone, or a pharmaceutically acceptable salt thereof, to the patient;
iii) measuring the serum creatinine level from the blood sample to provide a reference level;
iv) temporarily discontinuing administration of celivarone, or a pharmaceutically acceptable salt thereof;
v) obtaining a blood sample from the patient following discontinuance of administration of celivarone or a pharmaceutically acceptable salt thereof to the patient; and
vi) measuring the serum creatinine level in the blood sample from the patient following discontinuance to provide a reference level.

In some embodiments, the patient is also being treated with a compound selected from the group consisting of ACE-I, ARB and potassium sparing diuretics. In some embodiments, the serum creatinine level in step iii) is measured within 3 to 15 days, more particularly within 3 to 11 days after initiation of treatment with celivarone. In some embodiments, the serum creatinine level in step iii) is measured within one week after initiation of treatment with celivarone.

In an embodiment, the subject of the instant invention is a method of treatment of patients with arrhythmia, wherein celivarone or one of its pharmaceutically acceptable salts is used in combination with at least a compound B selected from ACE-I, ARB and potassium sparing diuretics, said method comprising:
i) initiating administration of celivarone, or a pharmaceutically acceptable salt thereof, in said patient,
ii) obtaining a blood sample from the patient following initiation of celivarone, or a pharmaceutically acceptable salt thereof, to the patient,
iii) measuring the serum creatinine level in the blood sample to provide a reference level,
iv) obtaining a blood sample from the patient and measuring serum creatinine level during treatment of the patient by celivarone, or a pharmaceutically acceptable salt thereof, and by the compound B,
v) determining the cause of an increase serum creatinine level above the reference level,
vi) continuing treatment of celivarone and compound B when the increase is due to treatment with celivarone or pharmaceutically acceptable salt thereof; or interrupting treatment with compound B when the increase is caused by treatment with compound B; or undertaking appropriate treatment when the increase is due to a cause other than administration of celivarone or of compound B, and
vii) repeating steps iv) to vii) at regular intervals for the duration of the treatment with celivarone and compound B.

In some embodiments, the serum creatinine level in step iii) is measured within 3 to 15 days, more particularly within 3 to 11 days after initiation of treatment with celivarone. In some embodiments, the serum creatinine level in step iii) is measured within one week after initiation of treatment with celivarone.

In an embodiment, the subject of the instant invention is a method of providing celivarone, or a pharmaceutically acceptable salt thereof, wherein said celivarone or pharmaceutically acceptable salt thereof is provided along with information indicating the serum creatinine level increase following treatment initiation of celivarone, or a pharmaceutically acceptable salt thereof. In some embodiments the information comprises printed matter that indicates that serum creatinine level increase following treatment initiation of celivarone, or a pharmaceutically acceptable salt thereof. In some embodiments, the information comprises written material recommending that if an increase in creatininemia is observed, this value should be used as the new baseline. In some embodiments, the printed matter is a label.

The term "providing" includes selling, distributing, shipping, offering for sell, importing, etc ...

In an embodiment, the subject of the instant invention is a method of promoting the use of celivarone or a pharmaceutically acceptable salt thereof, the method comprising the step of conveying to a recipient at least one message selected from the group consisting of:
(1) measure plasma creatinine values within about one week after treatment initiation of celivarone or a pharmaceutically acceptable salt thereof;
(2) if an increase in creatininemia is observed, this value should be used as the new baseline;
(3) serum creatinine level increase following treatment initiation of celivarone or a pharmaceutically acceptable salt thereof;
(4) the elevation of serum creatinine level reaches a plateau; and
(5) an increase in serum creatinine level is reversible after discontinuation of celivarone or pharmaceutically acceptable salt thereof.

In an embodiment, the subject of the instant invention is an article of manufacture comprising:
a) a packaging material;
b) celivarone or a pharmaceutically acceptable salt thereof; and
c) a label or package insert contained within the packaging material indicating that serum creatinine level may change following treatment initiation of celivarone, or a pharmaceutically acceptable salt thereof.

In an embodiment, the subject of the instant invention is a package comprising celivarone or a pharmaceutically acceptable salt thereof and a label, said label comprising a printed statement which informs a prospective user that an increase in serum creatinine has been observed following initiation of treatment with celivarone or a pharmaceutically acceptable salt thereof.

In an embodiment, the subject of the instant invention is a package comprising celivarone or a pharmaceutically acceptable salt thereof and a label, said label comprising a printed statement which recommends that if an increase in creatininemia is observed following treatment initiation of celivarone, or a pharmaceutically acceptable salt thereof, the increased value should be used as a baseline.

In an embodiment, the subject of the instant invention is a method of transforming a patient treated by an association of celivarone and at least one compound B selected from ACE-I, ARB and potassium sparing diuretics by managing the risk of an increase in the morbidity and/or mortality rate in said patient which method comprises:
i) initiating celivarone treatment,
ii) measuring serum creatinine level increases following celivarone treatment initiation, which provides a reference level,
iii) measuring that the increase reaches a plateau,
iv) temporarily discontinuing celivarone treatment, and
v) measuring that the increase is reversible after celivarone discontinuation.

A pharmaceutically acceptable salt of celivarone may be in particular the fumarate salt.

The uses described above, as for example use of celivarone or one of its pharmaceutically acceptable salts for preparing a medicament for the treatment of the above mentioned diseases, may be understood as use of a compound or an association for the preparation of a medicament for the treatment of the above mentioned diseases or as use of a compound or an association for the treatment of the above mentioned diseases or as a method for the treatment of the above mentioned diseases or as a compound or an association for the prevention or the treatment of the above mentioned diseases.

These compound and association may also be used for the prevention or the treatment of the above mentioned diseases.

## Claims

1. Use of celivarone or one of its pharmaceutically acceptable salts for preparing a medicament for the treatment of patients with arrhythmia, said patients having an increase of creatinine level due to celivarone administration, said creatinine level increase following celivarone treatment initiation, said creatinine level increase reaching a plateau and being used as a new baseline, and said creatinine level increase being reversible after celivarone discontinuation.

2. The use according to claim 1, wherein said use involves the following steps:
i) initiating celivarone treatment,
ii) measuring serum creatinine level increases following celivarone treatment initiation, which provides a reference level, said serum creatinine level reaching a plateau,
iii) discontinuing temporarily celivarone, and
iv) measuring that the increase is reversible after celivarone discontinuation.

3. Use of celivarone or one of its pharmaceutically acceptable salts for preparing a medicament for the treatment of patients with arrhythmia, said patients having an increase of creatinine level not due to administration of at least a compound B selected from selected from ACE-I, ARB and potassium sparing diuretics, wherein said use involves steps i) to iv) as defined in claim 2.

4. A method of managing the risk of an increase in the morbidity and/or mortality rate in patients treated by an association of celivarone and of at least a compound B selected from ACE-I, ARB and potassium sparing diuretics, which method comprises the steps i) to iv) as defined in claim 2.

5. Use of celivarone or one of its pharmaceutically acceptable salts for preparing a medicament for use in the treatment of arrhythmia, wherein celivarone or one of its pharmaceutically acceptable salts is used in combination with at least a compound B selected from ACE-I, ARB and potassium sparing diuretics and wherein said use involves the following steps:
a) initially measuring serum creatinine levels, which provides a reference level,
b) monitoring serum creatinine levels at regular intervals during treatment of the patient by celivarone and by the compound B,
c) if the serum creatinine levels increases above the reference level, determining if the increase is due to celivarone, to compound B or to another cause,
d) if the increase in the serum creatinine level above the reference level is due to celivarone, then the treatment with celivarone and compound B can be pursued; if the increase in the serum creatinine level above the reference level is due to compound B, then the treatment with compound B may be interrupted; if the increase in the serum creatinine level above the reference level is due to another cause than administration of celivarone or of compound B, then appropriate treatment of such another cause shall be undertaken,
e) repeating steps b), c) and d) for the duration of the treatment with celivarone and compound B.

6. The use according to claim 5, wherein patients have an increase of creatinine level due to celivarone administration, said patients being defined with the following steps:
a) initially measuring serum creatinine levels, which provides a reference level,
b) monitoring serum creatinine levels at regular intervals during treatment of the patient by celivarone and by the compound B,
c₁) if the serum creatinine level increases above the reference level, temporarily interrupting treatment with celivarone, while maintaining treatment with compound B,
d₁) when the increase is reversible, treatment with said combination is pursued, e₁) repeating steps b), c₁) and d₁) for the duration of the treatment with said combination.

7. The use according to claim 5, wherein patients have an increase of creatinine level due to compound B administration, said patients being defined with the following steps:
a) initially measuring serum creatinine levels, which provides a reference level,
b) monitoring serum creatinine levels at regular intervals during treatment of the patient by celivarone and by the compound B,
c₁) if the serum creatinine level increases above the reference level, temporarily interrupting treatment with celivarone, while maintaining treatment with compound B,
d₂) when the increase is not reversible, treatment with compound B may be interrupted,
e₂) repeating steps b), c₁) and d₂) for the duration of the treatment with said combination.

8. The use according to any of claims 5 to 7, wherein the serum creatinine level in step a) is measured within 3 to 15 days after initiation of treatment with celivarone.

9. The use according to any of claims 5 to 8, wherein the serum creatinine level in step a) is measured within one week after initiation of treatment with celivarone.

10. The use according to any of claims 5 to 9, wherein the monitoring of the serum creatinine level is effected according to the prescription labelling for the compound B.

11. The use according to claim 5, wherein step c) is achieved by temporarily interrupting treatment with celivarone, while maintaining treatment with compound B.

12. The use according to claim 11, wherein the interruption of celivarone treatment ranges from 1 to 2 weeks.

13. A method of managing the risk of an increase in the morbidity and/or mortality rate in patients treated by an association of celivarone and of at least a compound B selected from ACE-I, ARB and potassium sparing diuretics, which method comprises the steps a) to e) as defined in claim 5.

14. The method according to claim 13, for the treatment of patients with arrhythmia.

15. A method of treatment of patients with cardiac arrhythmia comprising the administration of celivarone to patients treated, in addition, by at least a compound B selected from ACE-I, ARB and potassium sparing diuretics, which method comprises the steps a) to e) as defined in claim 5.

16. A method of treatment of patients with arrhythmia comprising:
i) initiating administration of celivarone or a pharmaceutically acceptable salt thereof to said patient;
ii) obtaining a blood sample from the patient following initiation of administration of celivarone, or a pharmaceutically acceptable salt thereof, to the patient, and
iii) measuring the serum creatinine level from the blood sample to provide a reference level.

17. The method according to claim 18, further comprising:
iv) temporarily discontinuing administration of celivarone, or a pharmaceutically acceptable salt thereof, and
v) obtaining a blood sample from the patient following discontinuance of administration of celivarone or a pharmaceutically acceptable salt thereof to the patient, and
vi) measuring the serum creatinine level in the blood sample from the patient following discontinuance to provide a reference level.

18. The method according to claim 16 or claim 17, wherein the patient is also being treated with a compound selected from the group consisting of ACE-I, ARB and potassium sparing diuretics.

19. A method of treatment of patients with arrhythmia, wherein celivarone or one of its pharmaceutically acceptable salts is used in combination with at least a compound B selected from ACE-I, ARB and potassium sparing diuretics, said method comprising:
i) initiating administration of celivarone, or a pharmaceutically acceptable salt thereof, to said patient;
ii) obtaining a blood sample from the patient following initiation of celivarone, or a pharmaceutically acceptable salt thereof, to the patient,
iii) measuring the serum creatinine levels in the blood sample to provide a reference level,
iv) obtaining a blood sample from the patient and measuring serum creatinine levels during treatment of the patient by celivarone, or a pharmaceutically acceptable salt thereof, and by the compound B,
v) determining the cause of an increase serum creatinine levels above the reference level,
vi) continuing treatment of celivarone and compound B when the increase is due to treatment with celivarone or pharmaceutically acceptable salt thereof; or interrupting treatment with compound B when the increase is caused by treatment with compound B; or undertaking appropriate treatment when the increase is due to a cause other than administration of celivarone or of compound B, and
vii) repeating steps iv) to vii) at regular intervals for the duration of the treatment with celivarone and compound B.

20. The method according to any one of claims 16 to 19, wherein the serum creatinine level in step iii) is measured within 3 to 15 days after initiation of treatment with celivarone.

21. The method according to any one of claims 16 to 20, wherein the serum creatinine level in step iii) is measured within one week after initiation of treatment with celivarone.

22. A method of providing celivarone, or a pharmaceutically acceptable salt thereof, wherein said celivarone or pharmaceutically acceptable salt thereof is provided along with information indicating the serum creatinine levels increase following treatment initiation of celivarone, or a pharmaceutically acceptable salt thereof.

23. The method according to claim 22 wherein the information comprises printed matter that indicates that serum creatinine level increases following treatment initiation of celivarone, or a pharmaceutically acceptable salt thereof.

24. The method according to claim 22 or 23 wherein the information comprises written material recommending that if an increase in creatininemia is observed, this value should be used as the new baseline.

25. The method according to claim 23 or 24 wherein the printed matter is a label.

26. A method of promoting the use of celivarone or a pharmaceutically acceptable salt thereof, the method comprising the step of conveying to a recipient at least one message selected from the group consisting of:
(1) measure plasma creatinine values one week after treatment initiation of celivarone or a pharmaceutically acceptable salt thereof;
(2) if an increase in creatininemia is observed, this value should be used as the new baseline;
(3) serum creatinine levels increase following treatment initiation of celivarone or a pharmaceutically acceptable salt thereof;
(4) the elevation of serum creatinine level reaches a plateau; and
(5) an increase in serum creatinine levels is reversible after discontinuation of celivarone or pharmaceutically acceptable salt thereof.

27. An article of manufacture comprising
a) a packaging material;
b) celivarone or a pharmaceutically acceptable salt thereof; and
c) a label or package insert contained within the packaging material indicating that serum creatinine levels may change following treatment initiation of celivarone, or a pharmaceutically acceptable salt thereof.

28. A package comprising celivarone or a pharmaceutically acceptable salt thereof and a label, said label comprising a printed statement which informs a prospective user that an increase in serum creatinine has been observed following initiation of treatment with celivarone or a pharmaceutically acceptable salt thereof.

29. A package comprising celivarone or a pharmaceutically acceptable salt thereof and a label, said label comprising a printed statement which recommends that if an increase in creatininemia is observed following treatment initiation of celivarone, or a pharmaceutically acceptable salt thereof, the increased value should be used as a baseline.
